# EUROPÄISCHE PATENTSCHRIFT

(11) **EP 1 177 180 B1**
(45) Veröffentlichungstag und Bekanntmachung des Hinweises auf die Patenterteilung: **20.07.2005**
(21) Anmeldenummer: 00927036.4
(22) Anmeldetag: 25.04.2000
(51) Int. Cl.: C07D 251/52, D06L 3/12

(54) **STILBENAUFHELLER**
STILBENE BRIGHTENER
AGENT DE BLANCHIMENT OPTIQUE A BASE DE STILBENE

(30) Priorität: 05.05.1999 DE 19920784
(43) Veröffentlichungstag der Anmeldung: 06.02.2002
(73) Patentinhaber: LANXESS Deutschland GmbH, 51369 Leverkusen (DE)
(72) Erfinder: VOGT, Uwe, D-40789 Monheim (DE); BROCKMANN, Rolf, D-51469 Bergisch Gladbach (DE); ROICK, Thomas, D-51375 Leverkusen (DE); GASSEN, Karl-Rudolf, D-40885 Ratingen (DE); FELDHUES, Ulrich, Mount Pleasant, SC 29466 (US)
(86) Internationale Anmeldenummer: PCT/EP2000/003685
(87) Internationale Veröffentlichungsnummer: WO 2000/068211

(56) Entgegenhaltungen:
- EP-A- 0 024 380
- EP-A- 0 626 374
- EP-A- 0 860 437
- DE-A- 2 056 195
- DE-A- 2 335 570
- GB-A- 1 021 193
- US-A- 3 525 618

## Beschreibung

Die Erfindung betrifft neue Aufheller, ein Verfahren zur ihrer Herstellung sowie ihre Verwendung zum Aufhellen von Substraten.

Gemäß DE-A-2 335 570 werden Stilbenaufheller offenbart, deren endständige Triazinylreste mit kurzkettigen Polyetheralkoholen substituiert sind.

Gemäß EP-A-24 380 sind Stilbenaufheller bekannt, deren endständige Triazinylreste mit Phenyl gesättigten Ethylenoxideinheiten substituiert sind.

Überraschenderweise wurden nun optische Aufheller gefunden, die der Formel (I) entsprechen 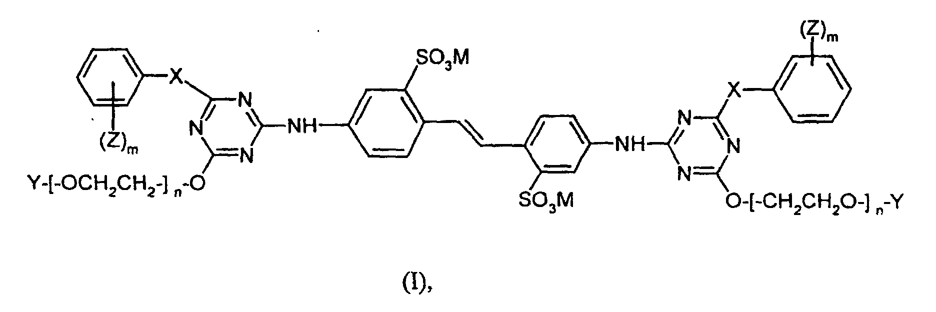 worin
- Z: SO₃M, COOR¹, CONR¹, SO₂NHR¹, NHCOR¹, COR² oder CN bedeutet, wobei
- R¹: für M oder C₁-C₃-Alkyl und
- R²: für C₁-C₃-Alkyl oder Phenyl stehen,

- X: O oder NR³ bedeutet, wobei
- R³: Wasserstoff oder C₁-C₄-Alkyl bedeutet,
- Y: Wasserstoff oder C₁-C₄-Alkyl, insbesondere Methyl bedeutet,
- M: Wasserstoff, Alkalimetallkation oder gegebenenenfalls substituiertes Ammoniumion bedeutet,
- n: für eine Zahl von 7 bis 25 und
- m: für 0, 1 oder 2 steht.

Der Index n soll im Rahmen dieser Anmeldung als statistische Größe verstanden werden, der vorzugsweise den Mittelwert repräsentiert.

Bevorzugte Aufheller sind solche in der sich der Rest der Formel 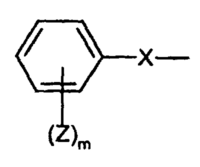 von

Verbindungen der Formel (V) 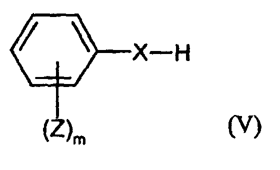 ableitet.

Als bevorzugte Verbindungen der Formel (V) kommen dabei in Frage: Phenol, Phenolsulfonsäure, Anilin, Sulfanilsäure, Dimetanilsäure (2,5-Disulfonsäureanilin).

Bevorzugte Aufheller der Formel (I) sind solche, worin
- Z: für SO₃M,
- m: für 0, 1 oder 2
- X: für NH,
- n: für 7 bis 15,
- Y: für Wasserstoff und
- M: für Wasserstoff, Na, K oder ein C₁-C₄-mono-, -di-, -tri- oder -tetra-Alkanolammonium steht.

Besonders bevorzugte Aufheller der Formel (I) sind solche, worin
- Z: für SO₃M,
- m: für 1,
- X: für NH,
- n: für 7 bis 15,
- Y: für Wasserstoff und
- M: für Na oder K
steht.

Besonders bevorzugte Aufheller der Formel (I) entsprechen der Formel (II) 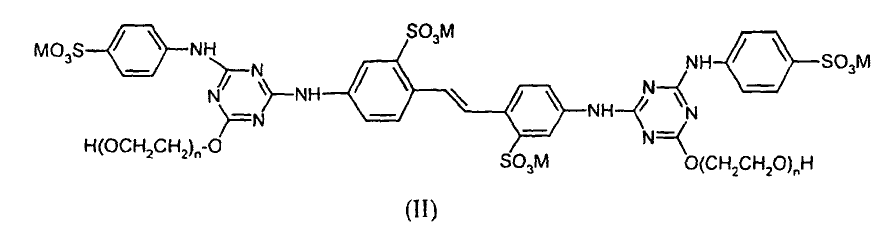 worin
- M: für Wasserstoff, Na oder K steht und
- n: eine Zahl von 7 bis 15 bedeutet.

Die Erfindung betrifft weiter ein Verfahren zur Herstellung der Verbindunngen der Formel (I), das dadurch gekennzeichnet ist, daß man Verbindungen der Formel (III) 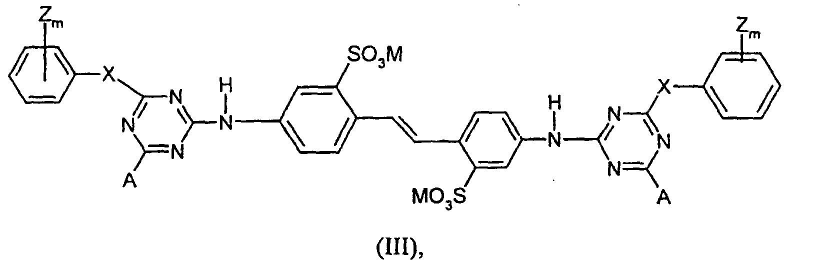 worin
- A: für Cl oder F, insbesondere für Cl, steht,
mit Polyglykolen bzw. Polyglykolethern der allgemeinen Formeln (IV)

Y-O-[CH₂CH₂O-]-ₙH (IV)

umsetzt,
wobei Z, X, Y, m und n die oben angegebenen Bedeutungen besitzen.

In einer besonderen Ausführungsform des erfindungsgemäßen Verfahrens erfolgt die Umsetzung bei einer Temperatur von 20 bis 100°C, vorzugsweise in Gegenwart eines säurebindenden Mittels, wie beispielsweise Natrium- oder Kaliumhydroxid oder -carbonat.

Die Umsetzung erfolgt im allgemeinen analog zu der in DE- 2.335 570 offenbarten Verfahrensweise.

Verbindungen der Formel (III) sind beispielsweise aus EP-A-860 437 bekannt.

Bevorzugte Verbindungen der Formel (IV) sind beispielsweise Polyethylenglykole, mit n = 7 bis 25, vorzugsweise mit einem mittleren Molekulargewicht von 200 bis 1000 g/mol, insbesondere 200, 300, 400, 600 und 1000 g/mol sowie deren entsprechenden Monomethylethern.

Besonders bevorzugt werden als Verbindungen der Formel (IV) Polyethylenglykole mit mittleren Molekulargewichten von 200 bis 600 g/mol eingesetzt.

In der Regel wird bei der Herstellung der erfindungsgemäßen Verbindungen der Formel (I) mit einem Überschuß an Verbindungen der Formel (IV) gearbeitet. Bevorzugt ist dabei ein molarer Überschuß von IV, bezogen auf III, von 5 bis 20 mol. Dieser Überschuß an (IV) kann z.B. durch Diafiltration aus dem Endprodukt entfernt werden. Bevorzugt verbleibt der Überschuß allerdings im Endprodukt, da er die Stabilität der Flüssigeinstellung als auch den Weißgrad des aufgehellten Materials positiv beeinflußt.

Die Erfindung betrifft dabei auch Aufhellerpräparationen, enthaltend den erfindungsgemäßen Aufheller der Formel (I) und ein Polyglykolether. Bevorzugte Polyglykolether sind solche der Formel (IV), wobei die Bedeutungen Y und n unabhängig von denen der Formel (I) sein können.

Bevorzugt sind derartige Aufhellerpräparationen enthaltend
10 - 25 Gew.-% Aufheller der Formel (I)
20 - 60 Gew.-% Polyglykolether
15 - 70 Gew.-% Wasser.

Zusätzlich können auch noch Salze enthalten sein.

Sofern Polyglykolether der Formel (IV) eingesetzt werden, können zusätzlich noch Polyethylenglykole (PEG) mit Molgewichten von 1500 bis 6000 g/mol eingesetzt werden. Letztere können von 0 bis 40 Gew.-%, bezogen auf die Präparation enthalten sein.

Die erfindungsgemäßen Verbindungen der allgemeinen Formel (I) stellen optische Aufhellungsmittel für verschiedene Substrate dar. Besonders bevorzugt sind als Substrate solche aus natürlicher Cellulose wie Baumwolle, Papier und Holzmassen in feiner Verteilung oder für Materialien aus regenerierter Cellulose, aus Wolle, oder synthetischen Polyamiden zu nennen. Die optisch aufzuhellenden Materialien können dabei in verschiedensten Verarbeitungszuständen wie Rohstoff, Halbfabrikat oder Fertigfabrikat und in den verschiedensten Verarbeitungsformen wie beispielsweise Fasern, Fäden, Gewebe, Gewirke, Vliese sowie Folien etc. vorliegen.

Den erfindungsgemäßen Verbindungen können auch Waschmittel zugefügt werden. Die zur Anwendung kommenden festen und flüssigen Waschmittel können die üblichen dem Stand der Technik entsprechenden Inhaltsstoffe enthalten.

Die erfindungsgemäßen Verbindungen lassen sich weiterhin während der Hochveredlung der Faserstoffe in Verbindung mit Kunstharzen und Kunstharzvorkondensaten aufbringen. Die Vernetzung der Kunstharze kann in einem weiten pH-Bereich, insbesondere von pH 1 bis pH 10, in üblicher Weise durchgeführt werden. So eignen sich insbesondere die erfindungsgemäßen Verbindungen der Formel (I) in denen
- Z: für SO₃M,
- m: für 1 oder 2,
- X: für NH,

- n: für 4 bis 15, vorzugsweise 5 bis 15, insbesondere 7 bis 15,
- Y: für Wasserstoff und
- M: für Na oder K
stehen, zum optischen Aufhellen von Cellulosematerialien aus sauren Vemetzungsbädem, wie es für die "wash and wear"-Ausrüstung von Cellulosefasern üblich ist.

Weiterhin können die erfindungsgemäßen Verbindungen dazu eingesetzt werden, den Sonnenschutzfaktor von textilen Materialien zu erhöhen. Der Einsatz von Diaminstilbendisulfonsäure-Derivaten zur Erhöhung des Sonnenschutzfaktors von textilen Materialien ist bekannt und z.B. in EP-A 728 749 (≙ GB 9 503 474) beschrieben. Für diesen Einsatz eignen sich insbesondere die erfindungsgemäßen Verbindungen der allgemeinen Formel (I), wobei
- Z: für COOR¹, mit R¹ C₁-C₃-Alkyl oder M, CONR¹, SO₂NHR¹, NHCOR¹, CO-R³, wobei R³ C₁-C₃-Alkyl oder Phenyl bedeutet, oder CN steht,
- m: für 1 steht,
- X: für NH steht,
- n: für 7 bis 15 steht,
- Y: für Wasserstoff oder Methyl steht,
- M: für Wasserstoff, ein Alkalimetallkation oder ein gegebenenfalls substituiertes Ammoniumion steht.

Zur Erhöhung des Sonnenschutzfaktors der textilen Materialien kann das textile Material direkt mit dem erfindungsgemäßen Verbindungen behandelt werden, oder aber der Effekt wird erzielt im Rahmen der normalen Haushaltswäsche, wenn das zum Einsatz kommende Waschmittel die erfindungsgemäße Verbindung enthält.

Die erfindungsgemäßen Aufheller der Formel (I) eignen sich zum Aufhellen von Papiermassen bei der Papierherstellung, z.B. Zellstoff, Holzstoff (chemical and mechanical pulp) und zum Aufhellen der in der Papierindustrie üblicherweise verwendeten Streichmassen und zwar zum Aufhellen von unpigmentierten, insbesondere aber von pigmentierten Papiermassen und Streichmassen.

Die bekannten Streichmassen enthalten als Bindemittel u.a. Kunststoffdispersionen auf Basis von Copolymerisaten aus Butadien-Styrol, Acrylnitril-Butadien-Styrol, Acrylsäureestern, Ethylen-Vinylchlorid oder Ethylen-Vinylacetat oder auf Basis von Homopolymerisaten, wie Polyvinylchlorid, Polyvinylidenchlorid, Polyethylen, Polyvinylacetat oder Polyurethanen. Ein bevorzugtes Bindemittel besteht aus Styrol-Butylacrylat- oder Styrol-Butadien-Acrylsäure-Mischpolymerisaten. Weitere Polymerlatices sind beispielsweise in US 3 265 654 beschrieben.

Zum Pigmentieren der Streichmassen dienen üblicherweise Aluminiumsilikate, wie China-Clay und Kaolin, ferner Bariumsulfat, Satinweiss, Titandioxid oder Calciumcarbonat (Kreide).

Die erfindungsgemäßen Streichmassen enthalten vorzugsweise 5 bis 70 Gew.-% eines Weißpigmentes. Das Bindemittel wird vorzugsweise in einer Menge verwendet, die ausreicht, daß der Trockengehalt an polymerer Verbindung 1 bis 30 Gew.-%, vorzugsweise 5 bis 25 Gew.-% des Weißpigmentes ausmacht. Die Menge des erfindungsgemäßen Aufhellers berechnet sich derart, daß der Aufheller in Mengen von 0,005 bis 1 Gew.-%, insbesondere 0,01 bis 0,55 Gew.-%, bezogen auf Weißpigment, vorliegt.

Die erfindungsgemäße Streichmasse kann dadurch hergestellt werden, daß man die Komponenten in beliebiger Reihenfolge bei Temperaturen von 10 bis 100°C, vorzugsweise 20 bis 80°C, mischt. Zu den Komponenten zählen hier auch die üblichen Hilfsmittel, die zur Regulierung der rheologischen Eigenschaften, wie Viskosität oder Wasserrückhaltevermögen, der Streichmassen eingesetzt werden können. Solche Hilfsmittel sind z.B. natürliche Bindemittel, wie Stärke, Casein, Protein oder Gelatine. Celluloseether, wie Carboxyalkylcellulose oder Hydroxyalkylcellulose, Alginsäure. Alginate, Polyethylenoxid oder Polyethylenoxidalkylether, Mischpolymerisate von Ethylenoxid und Propylenoxid, Polyvinylalkohol, Polyvinylpyrrolidon, wasserlösliche Kondensationsprodukte vom Formaldehyd mit Harnstoff oder Melamin, Polyphosphate oder polyacrylsaure Salze.

Die erfindungsgemäßen Aufheller der Formel (I) werden entweder in die fertige Streichmasse oder in eine der Komponenten der Streichmasse eingearbeitet.

Die erfindungsgemäße Streichmasse kann zum Beschichten von Papier, Holz, Folien, wie z.B. Cellulose, Cellulosetriacetat, Textilstoffen etc. verwendet werden. Besonders bevorzugt ist die Anwendung auf Papier und Karton sowie Photopapieren.

Die Streichmasse kann auf das Substrat durch jedes herkömmliche Verfahren aufgebracht werden, beispielsweise mit einem Luftmesser, einem Streichmesser, einer Bürste, einer Rolle, einer Rakel oder einem Stab, worauf dann die Beschichtung z.B. mit einem Infrarottrockner und/oder Heißlufttrockner bei Temperaturen der Substratoberfläche im Bereich von 70 bis 200°C, vorzugsweise von 90 bis 130°C, bis auf eine Restfeuchte von 3 bis 6 Gew.-% getrocknet wird.

Durch die Verwendung der erfindungsgemäßen Streichmassen zeichnen sich die erhaltenen Beschichtungen durch eine optimale Verteilung der optischen Aufheller über die gesamte Oberfläche und eine dadurch bedingte Steigerung des Weißgrades sowie eine hohe Lichtechtheit aus.

### Beispiele

### Beispiel 1: (Herstellung der Verbindung der Formel 1)

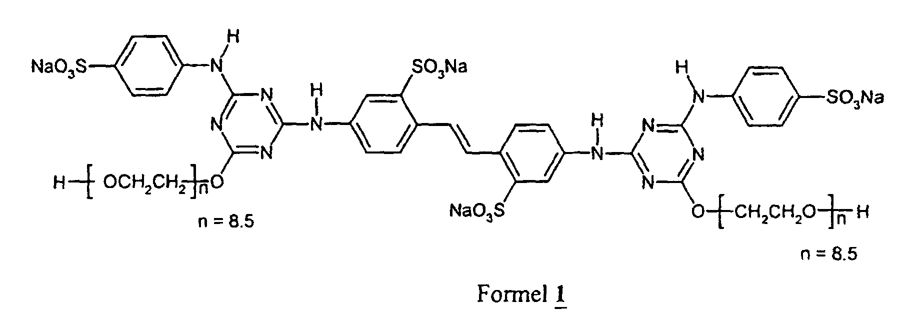

117 g der Verbindung der Formel Formel (2) 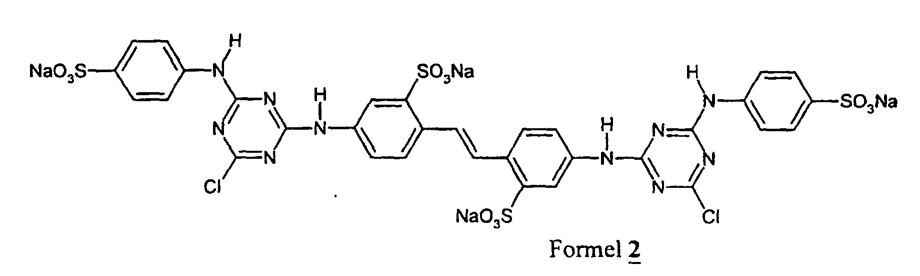 werden in einer Mischung aus 500 g Polyglykol 400 und 281 g 7%iger Natriumhydroxidlösung suspendiert. Es wird auf ca. 50°C erwärmt und bei dieser Temperatur 4 Stunden verrührt. Man läßt abkühlen und stellt durch Zugabe von 10%iger Salzsäure einen pH-Wert von etwa 7 ein. Die resultierende hellgelbe, klare Lösung kann direkt in die entsprechenden Anwendungen eingesetzt werden.

Durch mehrmalige Diafiltration kann die Aufhellerlösung allerdings auch von dem Überschuß Polyglykol 400 und dem bei der Reaktion entstandenen Salzen befreit werden. Nach Diafiltration und Trocknung der Lösung resultiert ein hellgelber Feststoff.

Aus der Elementaranalyse ergibt sich eine Summenforniel von C₆₆H₉₃N₁₀O_{32.5}S₄Na₄, entsprechend der Formel **1** x 1.5H₂O :

| | C | H | N | O | S | Na |
|---|---|---|---|---|---|---|
| gefunden | 44.9 | 5.2 | 7.9 | 29.9 | 7.3 | 5.01 |
| berechnet | 44.9 | 5.3 | 7.9 | 29.9 | 7.3 | 5.2 |

Die Startverbindung der Formel 2 wird in bekannter Weise durch Umsetzung von Cyanurchlorid mit 4,4'-Diaminostilben-2,2'-disulfonsäure und anschließender Umsetzung mit 4-Aminobenzolsulfonsäure hergestellt.

Die Verbindung der Formel 1 läßt sich mit gleichem Erfolg herstellen, indem man eine Suspension aus 117 g der Verbindung der Formel (2) in 281 g 7 %iger Natriumhydroxidlösung vorlegt und unter Rühren anschließend 500 g Polyglykol 400 eindosiert. Es folgt eine dreistündige Reaktionszeit bei 30°C. Durch Zugabe von Salzsäure wird der pH-Wert in den Neutralbereich gebracht.

Ebenfalls möglich ist es, eine Mischung aus 117 g der Verbindung der Formel (2), 500 g Polyglykol 400 und 332 g einer 15 %igen, wässrigen Natriumcarbonatlösung vorzulegen und anschließend 6 Stunden bei 100°C reagieren zu lassen. Nach dem Abkühlen stellt man den pH-Wert durch Zugabe von Salzsäure auf 7.

### Beispiel 2: (Herstellung einer Verbindung der Formel (3))

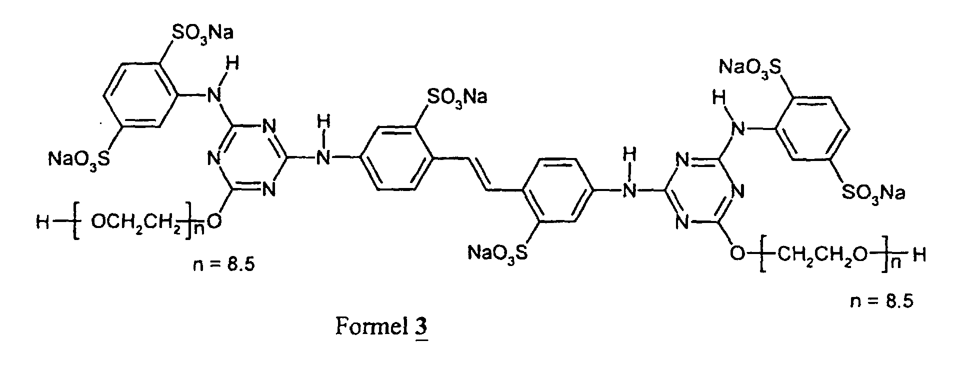

193 g der Verbindung der Formel **4** 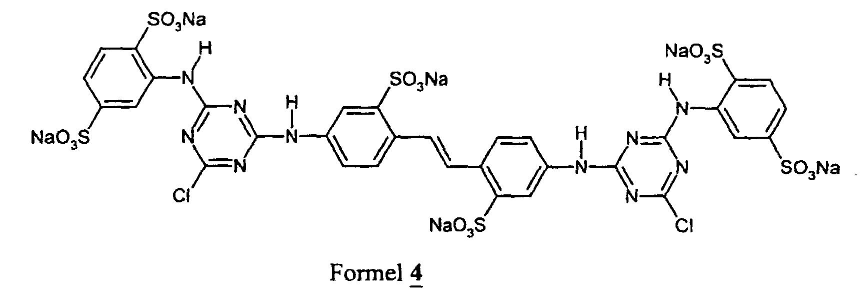 werden in einer Mischung aus 1000 g Polyglykol 400 und 360 g 5%iger Natriumhydroxidlösung suspendiert. Es wird auf circa 50°C erwärmt und bei dieser Temperatur 4 Stunden verrührt. Man läßt abkühlen und stellt durch Zugabe von 10%iger Salzsäure einen pH-Wert von etwa 7 ein.

Die resultierende, hellgelbe Lösung kann direkt in die entsprechenden Anwendungen eingesetzt werden.

Die Startverbindung der Formel 4 wird in bekannter Weise durch Umsetzung von Cyanurchlorid mit 4,4'-Diaminostilben-2,2'-disulfonsäure und 2,5-Disulfoaminobenzol hergestellt.

### Beispiel 3: (Anwendung im Papierstrich)

Ein aufheller- und holzfreies DIN A4 Rohpapier (Flächengewicht 80 g/m²) wird auf einem Laborrakelgerät (Firma Erichsen, K-Control-Coater, Modell K 202) mit einer Streichfarbe folgender Zusammensetzung bestrichen:
60 Teile Calciumcarbonat
40 Teile Kaolin
10 Teile SBR-Latex
1 Teil Polyvinylalkohol
0,25 Teile Polyacrylsäure

Der pH-Wert der Streichfarbe wird mit verdünnter Natronlauge auf 8-8,5 eingestellt. Der Feststoffgehalt der Streichfarbe wird durch Zusatz von Wasser auf 60 - 65% gebracht. Es folgt der Zusatz von 11 g bezogen auf 1 kg Streichfarbe von Verbindung aus Beispiel 1.

Die Blätter werden 1 min. bei 95°C im Trockenzylinder getrocknet und danach für 3 Stunden bei 23°C und einer Luftfeuchtigkeit von 50 % gelagert, bevor sie vermessen werden. Es resultiert ein Papier mit einem sehr guten Weißgrad.

### Beispiel 4: (Anwendung in der Papier-Leimpresse)

Ein aufheller- und holzfreies DIN A4 Rohpapier (Flächengewicht 80 g/m²) wird auf einer Laborleimpresse (Firma Werner Matthis AG, TYP-Nr. HF 18374) mit einer wässrigen Flotte benetzt, die
50 g/l Stärke und
1 g/l Verbindung aus Beispiel 1 enthält.

Der pH-Wert der Flotte beträgt 7. Die Naßaufnahme beträgt dabei ca. 50-60 %.

Anschließend werden die Blätter 1 min bei 95°C im Trockenzylinder getrocknet und danach für 3 Stunden bei 23°C und einer Luftfeuchtigkeit von 50 % gelagert, bevor sie vermessen werden. Es resultiert ein Papier mit einem sehr guten Weißgrad.

### Beispiel 5: (Anwendung auf Baumwolle, Klotz-Trocknungsverfahren)

Abgekochte und gebleichte Baumwollwebware wird auf dem Labor-Foulard mit einer wässrigen Flotte geklotzt, welche
14 g/l Verbindung aus Beispiel 1 und
3 g/l Natriumsulfat enthält.

Die Flottenaufnahme des Gewebes wird durch Abquetschen zwischen den Foulardwalzen auf ca. 80% eingestellt. Sofort anschließend erfolgt die Trocknung der Ware durch eine Spannrahmenpassage bei 100°C für 30 Sekunden. Durch diese Behandlung wird auf der Ware ein sehr guter Aufhelleffekt erzielt.

### Beispiel 6: (Anwendung auf Baumwolle, Trockenvernetzung)

Abgekochte und gebleichte Baumwollpopeline wird auf dem Labor-Foulard mit einer wässrigen Flotte folgender Zusammensetzung imprägniert:
14 g/l Verbindung aus Beispiel 1
80 g/l Fixapret® NF (Produkt der BASF)
25 g/l Condensol® N (Produkt der BASF)

Die Ware wird zwischen Walzen auf eine Flottenaufnahme von ca. 80% des Trockengewichtes abgequetscht. Anschließend erfolgt die Trocknung auf dem Spannrahmen bei 100°C für 30 Sekunden. Kondensiert wird ebenfalls auf dem Spannrahmen bei 150°C für 4 Minuten.

Durch diese Behandlung wird auf der Ware ein sehr guter Aufhelleffekt erzielt.

### Beispiel 7: (Vergleichsbeispiel zum Stand der Technik im Papierstrich)

Gemäß der in Beispiel 3 beschriebenen Art werden folgende beiden Verbindungen miteinander verglichen:
a) Stand der Technik: (DE-A-23 35 570) 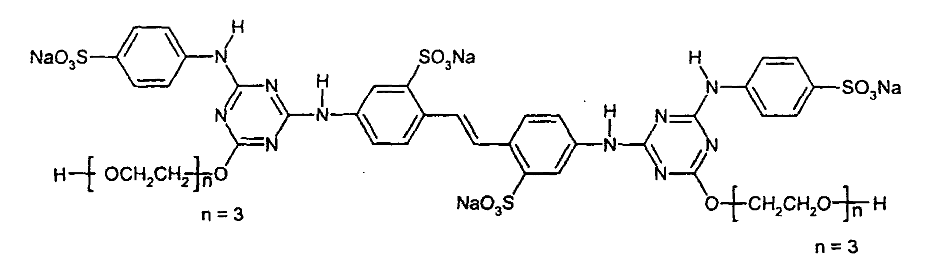
b) Erfindungsgemäße Verbindung aus Beispiel 1:

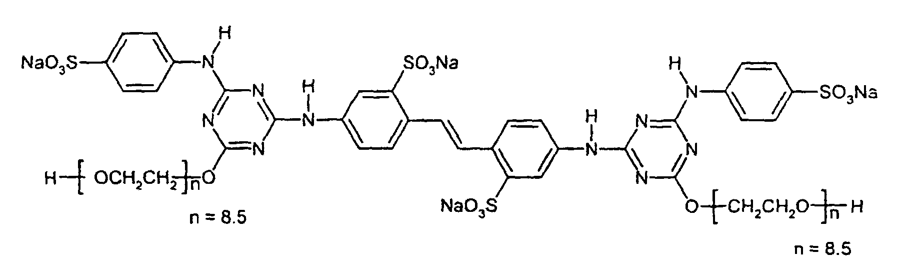

Beide Proben werden zuvor durch Wasserzusatz auf gleiche Farbstärke gebracht und anschließend mit jewiels 34% Polyethylenglykol 1500 versetzt. Um den Weißaufbau zu beurteilen, werden die beiden Proben in verschiedenen Konzentrationen eingesetzt, und zwar jeweils 0.4%, 0.8%, 1.6%, 2.4% und 3.2% bezogen auf den Feststoffgehalt der Streichfarbe.

Die Tabelle zeigt die Ergebnisse der Weißgradmessung von den so hergestellten Papieren:

| Weißgrad nach CIELAB | Einsatz | | | | |
|---|---|---|---|---|---|
| | 0.4 % | 0.8 % | 1.6 % | 2.4 % | 3.2 % |
| a) Stand der Technik | 81.2 | 85.4 | 92.0 | 98.1 | 101.6 |
| b) Erfindungsgemäße Probe | 81.4 | 86.9 | 94.4 | 100.0 | 103.6 |

Man erkennt den erhöhten Weißgrad, der durch Einsatz der erfindungsgemäßen Verbindung im Vergleich zum Stand der Technik erzielt wird.

## Patentansprüche

1. Verbindungen der Formel (I) 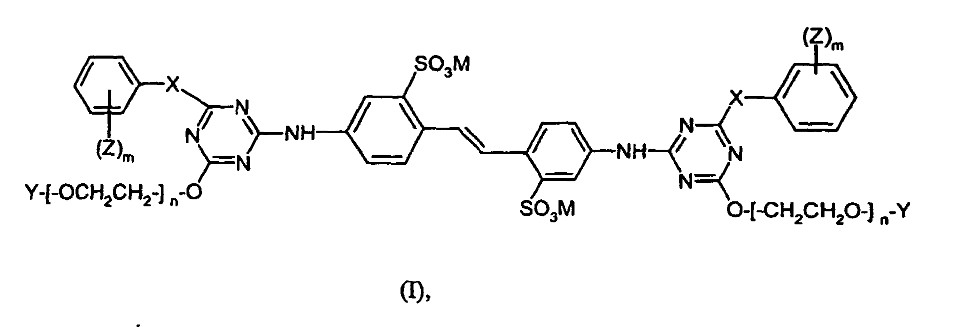 worin
Z SO₃M, COOR¹, CONR¹, SO₂NHR¹, NHCOR¹, COR² oder CN bedeutet, wobei
R¹ für M oder C₁-C₃-Alkyl und
R² für C₁-C₃-Alkyl oder Phenyl stehen,
X O oder NR³ bedeutet, wobei
R³ Wasserstoff oder C₁-C₄-Alkyl bedeutet,
Y Wasserstoff oder C₁-C₄-Alkyl, insbesondere Methyl bedeutet,
M Wasserstoff, Alkalimetallkation oder gegebenenenfalls substituiertes Ammoniumion bedeutet,
n für eine Zahl von 7 bis 25 und
m für 0, 1 oder 2 steht.

2. Verbindungen der Formel (I) gemäß Anspruch 1, worin sich der Rest der Formel 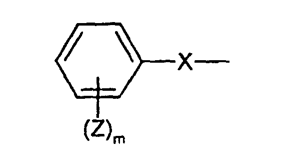 von Verbindungen der Formel (V) 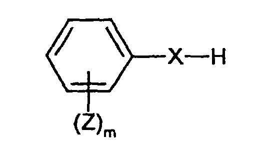 aus der Gruppe bestehend aus: Phenol, Phenolsulfonsäure, Anilin, Sulfamidsäure und Dimetanilsäure ableitet.

3. Verbindungen der Formel (I) gemäß Anspruch 1, **dadurch gekennzeichnet, daß**
Z für SO₃M,
m für 0, 1 oder 2
X für NH,
n für 7 bis 15,
Y für Wasserstoff und
M für Wasserstoff, Na, K oder ein C₁-C₄-mono, di, tri- oder tetra-Alkanolammonium steht.

4. Verbindungen der Formel (I) gemäß Anspruch 1, **dadurch gekennzeichnet, daß**
Z für SO₃M,
m für 1,
X für NH,
n für 7 bis 15,
Y für Wasserstoff und
M für Na oder K
steht.

5. Verbindungen der Formel (I) gemäß Anspruch 1, **dadurch gekennzeichnet, daß** sie der Formel (II) entspricht 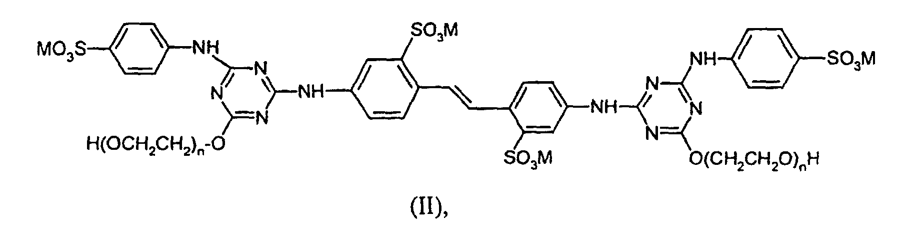 worin
M für Wasserstoff, Na oder K steht und
n eine Zahl von 7 bis 15 bedeutet.

6. Verfahren zur Herstellung von Verbindungen gemäß Anspruch 1, **dadurch gekennzeichnet, daß** man Verbindungen der Formel (III) 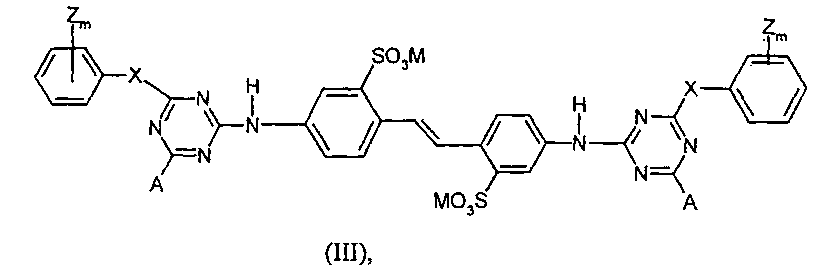 worin
A für Cl oder F steht, insbesondere für Cl,
mit Verbindungen der Formel (IV)
Y-O-[CH₂CH₂O-]-ₙH (IV)
umsetzt wobei M, Z, Y, X, m und n die in Anspruch 1 angegebene Bedetung haben.

7. Aufhellerpräparationen, enthaltend wenigstens einen Aufheller gemäß Anspruch 1 und ein Polyglykolether.

8. Streichmasse, enthaltend 5 bis 70 Gew.-% eines Weißpigmentes und 0,005 bis 1 Gew.-% eines Aufhellers, bezogen auf Weißpigment, der Formel (I) gemäß Anspruch 1.

9. Verwendung der Verbindung gemäß Anspruch 1 zum Aufhellen von Polyamid, Cellulose, Papier und Waschmittel.

## Claims

1. Compounds of the formula (I) 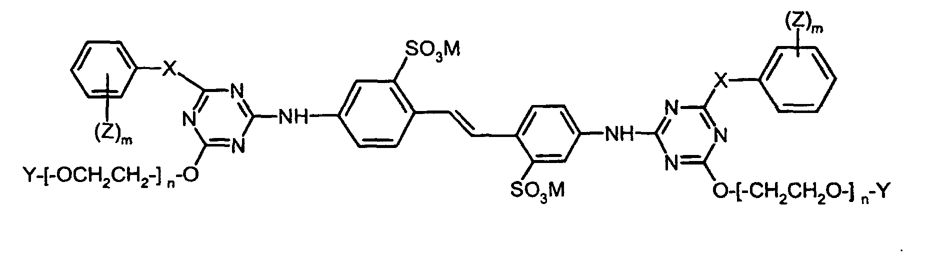 where
Z is SO₃M, COOR¹, CONR¹, SO₂NHR¹, NHCOR¹, COR² or CN, where
R¹ is M or C₁-C₃-alkyl and
R² is C₁-C₃-alkyl or phenyl,
X is O or NR³, where
R³ is hydrogen or C₁-C₄-alkyl,
Y is hydrogen or C₁-C₄-alkyl, especially methyl,
M is hydrogen, an alkali metal cation or an optionally substituted ammonium ion,
n is a number from 7 to 25 and
m is 0, 1 or 2.

2. Compounds of the formula (I) as claimed in claim 1 wherein the radical of the formula 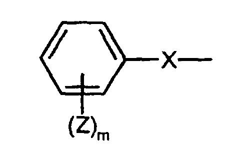 is derived from compounds of the formula (V) 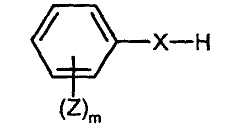 from the group consisting of phenol, phenolsulphonic acid, aniline, sulphamidic acid and dimetanilic acid.

3. Compounds of the formula (I) as claimed in claim 1, **characterized in that**
Z is SO₃M,
m is 0, 1 or 2
X is NH,
n is from 7 to 15,
Y is hydrogen and
M is hydrogen, Na, K or a C₁-C₄-mono-, -di-, -tri- or -tetra-alkanolammonium.

4. Compounds of the formula (I) as claimed in claim 1, **characterized in that**
Z is SO₃M,
m is 1,
X is NH,
n is from 7 to 15,
Y is hydrogen and
M is Na or K.

5. Compounds of the formula (I) as claimed in claim 1, **characterized in that** if conforms to the formula (II) 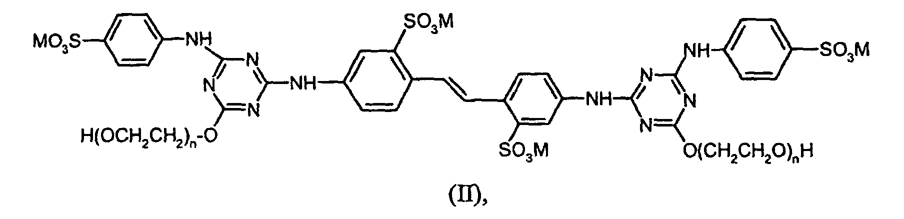 where
M is hydrogen, Na or K and
n is from 7 to 15.

6. Process for preparing compounds as claimed in claim 1, **characterized in that** compounds of the formula (III) 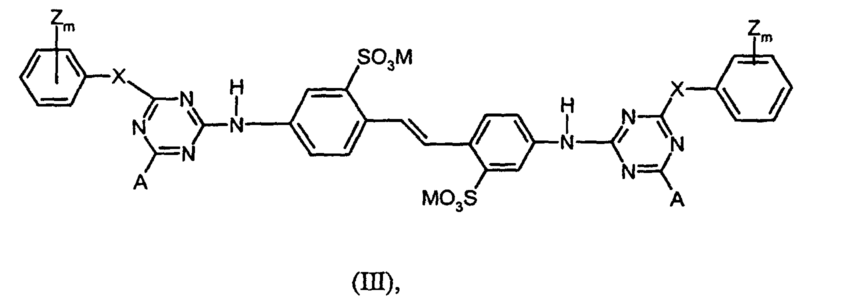 where
A is Cl or F, especially Cl,
are reacted with compounds of the formula (IV)
Y-O-[CH₂CH₂O-]-ₙH (IV)
where M, Z, Y, X, m and n are each as defined in claim 1.

7. Brightener preparations containing at least one brightener as claimed in claim 1 and a polyglycol ether.

8. Coating composition containing 5 to 70% by weight of a white pigment and 0.005 to 1% by weight of a brightener, based on white pigment, of the formula (I) as claimed in claim 1.

9. Use of the compound of claim 1 for brightening polyamide, cellulose, paper and laundry detergent.

## Revendications

1. Composés de formule (I) 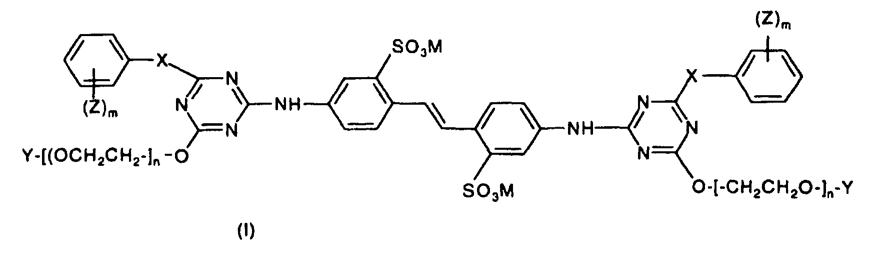 dans laquelle
Z représente SO₃M, COOR¹, CONR¹, SO₂NHR¹, NHCOR¹, COR² ou CN,
R¹ représente M ou un groupe alkyle en C₁ à C₃ et
R² représente un groupe alkyle en C₁ à C₃ ou phényle,
X représente O ou NR³,
R³ représentant l'hydrogène ou un groupe alkyle en C₁ à C₄,
Y représente l'hydrogène ou un groupe alkyle en C₁ à C₄, plus spécialement un groupe méthyle,
M représente l'hydrogène, un cation de métal alcalin ou un ion ammonium éventuellement substitué,
n est un nombre allant de 7 à 25 et
m est égal à 0, 1 ou 2.

2. Composés de formule (I) selon la revendication 1, pour lesquels le groupe de formule 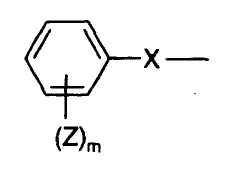 dérive de composés de formule (V) 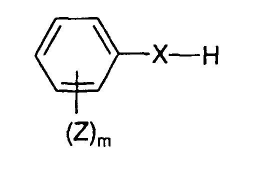 du groupe consistant en : le phénol, l'acide phénolsulfonique, l'aniline, l'acide sulfamique et l'acide dimétanilique.

3. Composés de formule (I) selon la revendication 1,
**caractérisés en ce que**
Z représente SO₃M,
m est égal à 0, 1 ou 2,
X représente NH,
n est un nombre allant de 7 à 15,
Y représente l'hydrogène et
M représente l'hydrogène, Na, K ou un ion mono-, di-, tri- ou tétra- (alcanol en C₁ à C₄)ammonium.

4. Composés de formule (I) selon la revendication 1,
**caractérisés en ce que**
Z représente SO₃M,
m est égal à 1
X représente NH,
n est un nombre allant de 7 à 15,
Y représente l'hydrogène et
M représente Na ou K.

5. Composés de formule (I) selon la revendication 1, **caractérisés en ce qu'**ils répondent à la formule (II) 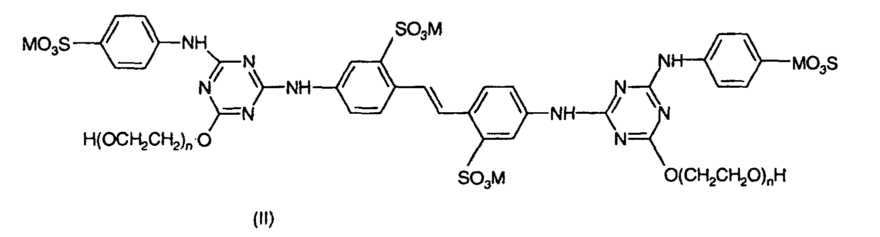 dans laquelle
M représente l'hydrogène, Na ou K et
n est un nombre allant de 7 à 15.

6. Procédé pour la préparation des composés selon la revendication 1, **caractérisé en ce que** l'on fait réagir des composés de formule (III) 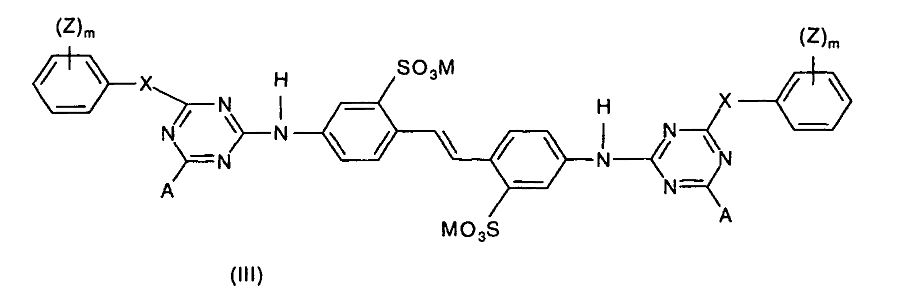 dans laquelle
A représente Cl ou F, plus spécialement Cl
avec des composés de formule (IV)
Y-O-[CH₂CH₂O-]-ₙH (IV)
M, Z, Y, X, m et n ayant les significations indiquées dans la revendication 1.

7. Compositions azurantes contenant au moins un azureur selon la revendication 1 et un éther de polyglycol.

8. Masse d'enduction contenant 5 à 70 % en poids d'un pigment blanc et un azureur de formule (I) selon la revendication 1 en proportion de 0,005 à 1 % du poids du pigment blanc.

9. Utilisation d'un composé selon la revendication 1 pour l'azurage des polyamides, de la cellulose, du papier et des produits de lavage.
